# FASCICULE DE BREVET EUROPEEN

(11) **EP 0 975 322 B1**
(45) Date de publication et mention de la délivrance du brevet: **13.03.2002**
(21) Numéro de dépôt: 98920593.5
(22) Date de dépôt: 08.04.1998
(51) Int. Cl.: A61K 7/48, A61K 7/06

(54) **UTILISATION D'UNE FRACTION PROTEIQUE EXTRAITE DE GRAINES D'HIBISCUS ESCULENTUS EN COSMETIQUE**
VERWENDUNG EINER AUS HIBISKUS-ESCULENTUS-SAMEN EXTRAHIERTEN PROTEINFRAKTION IN DER KOSMETIK
USE IN COSMETICS OF A PROTEIN FRACTION EXTRACTED FROM OKRA SEEDS

(30) Priorité: 16.04.1997 FR 9704853
(43) Date de publication de la demande: 02.02.2000
(73) Titulaire: Cognis France S.A., 31360 Saint Martory (FR)
(72) Inventeur: PAULY, Gilles, F-54000 Nancy (FR)
(74) Mandataire: Nuss, Pierre
(86) Numéro de dépôt international: FR9800715
(87) Numéro de publication internationale: WO9846205

(56) Documents cités:
- FR-A- 2 679 443
- BRYANT ET AL.: "processing, functional, and nutritional properties of okra seed products" JOURNAL OF FOOD SCIENCE, vol. 53, no. 3, 1988, pages 810-816, XP002049248 cité dans la demande
- DATABASE WPI Week 8327 Derwent Publications Ltd., London, GB; AN 83-702268 XP002049249 & JP 58 088 305 A (NIKKO CHEM CO)

## Description

La présente invention concerne le domaine de la cosmétologie, notamment les applications cutanées et capillaires, et a pour objet l'utilisation d'au moins une fraction protéique extraite d'Hibiscus esculentus ainsi qu'une composition comportant au moins un tel extrait.

L'Hibiscus esculentus (Abelmoshus esculentus ou okra - famille des Malvacées) est une plante d'origine africaine, introduite aux Etats-Unis et aux Indes de l'Est sous le nom espagnol de gumbo. C'est une des espèces botaniques cultivée pour ses gousses depuis plus de 2000 ans.

L'okra pousse dans de nombreuses régions du monde, telles que l'Inde, la Malaisie, les Philippines, l'Amérique (Middle-West), les régions méditerranéennes, l'Afrique et, plus généralement, au niveau des régions tropicales.

Les fruits (gousses), consommés à l'état jeune comme légumes, sont longs, verts et effilés ; ils présentent une saveur délicate et une texture interne mucilagineuse.

En dehors des gousses, intéressantes par le mucilage qu'elles contiennent, les graines d'Hibiscus esculentus ont également fait l'objet d'études afin d'étudier leur potentiel comme source nouvelle de protéines.

A cet effet, la composition chimique de la graine entière de différentes variétés d'okra (enveloppe + endosperme) a été déterminée.

De même, les propriétés (solubilité protéique, composition en acides aminés, capacité d'émulsion, capacité de moussage, valeur nutritionnelle) de différents produits obtenus à partir des graines (farine entière préparée à partir de graines dépelliculées, farine délipidée, concentré et isolat protéique) ont été étudiés dans un but alimentaire (voir par exemple BRYANT LA, MONTECALVO J, MOREY KS, LOY B : "Processing, functional and nutritional properties of Okra seed products", Journal of food science, vol 53 n° 3, 818-816).

La graine d'Okra contient par rapport à la matière sèche principalement les substances suivantes en % en poids :
- 17,7 à 21,8 % de protéines,
- 14,7 à 20,06 % de lipides,
- 4,33 à 4,62 % de cendres,
- 6,84 à 7,92 % d'eau,
- 0,0032 % de gossypol,
et notamment à l'état de de traces, les substances suivantes :
- Calcium : 282,26 mg/100g,
- Fer : 10,26 mg/100g,
- Thiamine : 0,69 mg/100g,
- Riboflavine : 0,14 mg/100g,
- Niacine : 4,01 mg/100g,
- α-tocophérol : 30,4 mg/100g.

(Voir à ce sujet KARAKOLTSIDIS PA, CONSTANTIDINES SM : "Okra seed : a new protein source", J. Agic Food Chem., 1975, 23 n° 6, 1204-1207 / WANDAWI AL : "Chemical composition of seeds of two okra cultivars Abelmoschus esculentus", Journal of agricultural and food chemistry, 1983, 31 n° 6, 1355-1358).

La composition en acides aminés des protéines de la graine d'Hibiscus esculentus concentrés ou des isolats protéiques est voisine de celle des protéines de soja et proche de celle de la caséine (voir tableau ci-dessous).

| Acide aminé g/16 g d'azote | Hibiscus (graines) KARAKOLTSIDIS et al MANDAWI AL | Hibiscus (graines) BRYANT et al | Concentré protéique BRYANT LA et al | Isolat protéique BRYANT LA et al | Soja (graine) KARAKOTSIDIS et al | Caséine KARAKOTSIDIS et al |
|---|---|---|---|---|---|---|
| Asp | 11,82 à 15,47 | 11,57 | 10,89 | 12,12 | 17,00 | 7,11 |
| Thr | 3,02 à 4,38 | 2,86 | 3,37 | 2,90 | 5,47 | 4,65 |
| Ser | 5,25 à 6,71 | 5,07 | 5,23 | 4,97 | 7,42 | 6,02 |
| Glu | 20,48 à 22,08 | 15,91 | 19,15 | 17,35 | 21,05 | 21,19 |
| Pro | 3,83 à 6,06 | 3,79 | 4,88 | 4,98 | 7,71 | 11,54 |
| Gly | 5,79 à 6,66 | 4,78 | 7,77 | 4,16 | 4,32 | 1,97 |
| Ala | 5,89 à 6,66 | 4,83 | 4,53 | 4,59 | 6,13 | 3,07 |
| Val | 4,0 à 6,4 | 4,24 | 4,42 | 4,33 | 5,26 | 6,72 |
| Cys | 1,54 à 2,53 | 3,63 | 1,89 | 1,90 | 1,61 | 0,36 |
| Met | 1,29 à 1,85 | 1,83 | 2,18 | 2,21 | 1,25 | 2,78 |
| Ile | 3,15 à 4,65 | 2,96 | 3,06 | 3,13 | 4,46 | 5,40 |
| Leu | 6,68 à 8,47 | 6,21 | 6,96 | 6,97 | 9,35 | 9,49 |
| Tyr | 3,6 à 3,83 | 3,46 | 5,15 | 4,03 | 3,72 | 5,81 |
| Phe | 3,93 à 4,7 | 4,41 | 4,80 | 4,85 | 5,26 | 5,23 |
| Lys | 7,24 à 8,9 | 6,22 | 6,19 | 6,47 | 8,00 | 8,80 |
| His | 1,78 à 2,99 | 2,34 | 3,61 | 3,83 | 2,67 | 2,91 |
| Arg | 11,04 à 12,46 | 11,17 | 10,02 | 9,98 | 10,07 | 3,74 |
| Trp | 0,85 à 0,96 | 2,02 | 2,57 | 2,03 | nd | nd |

Par rapport à la caséine, on note notamment des teneurs voisines en thréonine, en sérine, en acide glutamique, en valine, en isoleucine, en leucine, en phénylalanine, en lysine et en histidine.

Les différents travaux précités mettent donc en évidence l'intérêt des graines d'Hibiscus esculentus comme sources potentielles de protéines d'un point de vue alimentaire.

Par ailleurs, on connaît également l'utilisation à des fins dermatologiques d'un produit liquide visqueux obtenu par extraction à chaud et/ou sous pression des fruits d'Hibiscus esculentus (FR-A-2 679 443), ainsi que l'utilisation d'un matériau poudreux composé de polysaccharide extrait de graines immatures d'Hibiscus esculentus dans un produit cosmétique (JP-A-57/199969).

Enfin, par le document "Processing, functional and nutritional properties of okra seed products", Journal of food science, vol. 53, 3, 1988, pages 810 à 816, on connaît un mode d'obtention d'un extrait protéinique de graines d'Hibiscus esculentus.

Ce document présente également différents résultats de tests relatifs à des propriétés physico-chimiques de tels extraits, en comparaison avec des extraits de graines de soja, ce en vue d'une éventuelle utilisation alimentaire.

Or, les inventeurs ont constaté qu'il était possible d'utiliser directement des extraits de graines d'Hibiscus esculentus en cosmétique et que l'utilisation d'au moins une fraction protéique, préférentiellement soluble, extraite de graines d'Hibiscus esculentus ou Okra, notamment en tant que substitut de caséine, dans une composition ou un produit cosmétique permettait d'obtenir une composition ou un produit présentant des propriétés spécifiques surprenantes et avantageuses.

Ainsi, il a été constaté un fort pouvoir nutritif cellulaire, un effet adoucissant et biofilmogène, des effets conditionneurs, restructurants et réparateurs, ainsi que des effets anti-irritant, photoprotecteur, apaisant et anti-vieillissement cutané.

Les extraits précités peuvent être utilisés non seulement pour des applications de soins et d'hygiène de la peau (produits pour le visage ou pour le corps, produits de jour ou de nuit, produits solaires, produits d'hygiène anti-rides, produits amincissants), mais également dans le domaine des soins et de l'hygiène capillaires (lotions ou shampooings ; crèmes ; mousses ; produits protecteurs, réparateurs, adoucissants, filmogènes et photoprotecteurs ; produits pour permanentes et de coloration).

La préparation des protéines peut être réalisée par les techniques conventionnelles d'extraction des protéines végétales et de préparation des concentrés ou isolats protéiques, connus de l'homme de l'art et décrits notamment dans l'article de BRYANT LA, MONTECALVO J, MOREY KS et LOY B précité.

La matière première est constituée par des graines ou de la farine de graines d'Hibiscus esculentus (teneur en protéines = 21,6 % par rapport à la matière sèche).

La farine ainsi obtenue peut être délipidée par extraction dans de l'héxane à 45° C (avantageusement 3 extractions successives).

Le rendement en huile est de 16,2 à 23,9 % en poids selon que l'on part d'une farine de graines "totale" ou d'une farine de graines partiellement débarrassée des enveloppes ou coques par tamisage.

Les farines délipidées et partiellement débarrassées des coques des graines contiennent entre 37 et 44 % en poids de protéines (N x 6,25).

A titre d'exemples illustratifs et non limitatifs, on décrira ci-après différents procédés d'obtention et de préparation d'extraits de graines d'Hibiscus esculentus ou Okra.

### Exemple 1 :

On ajoute 100 g de farine enrichie (débarrassée partiellement des débris de coques) non délipidée dans un litre des milieux suivants :
- eau distillée,
- eau distillée contenant 1 g/l de NaCl,
- eau distillée contenant 5 g/l de NaCl,
- eau distillée contenant 10 g/l de NaCl.

Après 15 minutes d'agitation, le pH de la solution est ajusté à 9 avec NaOH 4 N.

L'extraction est menée pendant une heure et demi à température ambiante en maintenant le pH à 9.

Après centrifugation, la couche lipidique supérieure est éliminée et le surnageant aqueux est recueilli.

Le surnageant de couleur jaune d'or est ajusté à pH = 7,5, puis filtré jusqu'à 0,22 µm ; on note que plus la teneur en sels du solvant est importante, plus la filtration est facile.

La teneur en protéines du filtrat est déterminée par la méthode du Biuret. Les surnageants restent opalescents.

On obtient les résultats suivants :

| Solvant d'extraction | Protéines dans l'extrait filtré Biuret (g/l) |
|---|---|
| Eau distillée | 16,9 |
| NaCl 1 g/l | 13,3 |
| NaCl 5 g/l | 9,5 |
| NaCl 10g/l | 10,2 |

### Exemple 2 :

On ajoute dans 250 ml d'eau distillée contenant 5 g/l de chlorure de sodium, 25 g de farine délipidée enrichie (débarrassée des débris des coques).

Après 15 minutes d'agitation, le pH de la solution est ajusté selon l'essai aux pH suivants : 6-6,5-7-7,5.

L'extraction est menée pendant une heure à température ambiante.

Après centrifugation, le surnageant est récupéré puis filtré sur 5µm.

On obtient les résultats suivants :

| pH | Protéines Biuret (g/l) | Protéines (N x 6,25) (g/l) |
|---|---|---|
| 6 | 12,5 | 13,8 |
| 6,5 | 13,3 | 14,8 |
| 7 | 14,6 | 15,1 |
| 7,5 | 15,5 | 16,1 |

### Exemple 3 :

On ajoute, dans 250 ml d'eau distillée contenant 5g/l de chlorure de sodium, 25 g de farine délipidée enrichie.

Après 15 minutes d'agitation, le pH de la solution est ajusté à 8.

L'extraction est menée pendant 6 heures à température ambiante.

Après centrifugation, le surnageant est récupéré et le pH ajusté à 7,5, puis la solution est filtrée sur 5µm.

On obtient les résultats suivants :

| Durée (heures) | Protéines Biuret (g/l) |
|---|---|
| 2 | 18,45 |
| 4 | 20,3 |
| 6 | 19,7 |

### Exemple 4 :

On ajoute dans 250 ml d'eau distillée contenant 5 g/l de chlorure de sodium, 25 g de farine délipidée enrichie.

Après 15 minutes d'agitation, le pH de la solution est ajusté à pH 7,5.

L'extraction est menée pendant 6 heures à 45° C.

Après centrifugation, le surnageant est récupéré, son pH est ajusté à 7,5, puis la solution est filtrée sur 5µm.

On obtient les résultats suivants :

| Durée (heures) | Protéines Biuret (g/l) |
|---|---|
| 2 | 18,0 |
| 4 | 18,1 |
| 6 | 13,8 |

### Exemple 5 :

On ajoute dans 31 d'eau distillée contenant 5 g/l de chlorure de sodium, 300 g de farine délipidée enrichie.

Après 15 minutes d'agitation, le pH de la solution est ajusté à pH 7,5.

L'extraction est menée pendant 2 heures à 50° C.

Après centrifugation, le surnageant est récupéré puis filtré sur 5µm.

On obtient les résultats suivants :
Protéines Biuret : 13,7 g/l
Protéines kjeldahl : 14,6 g/l

Un litre de surnageant est prélevé et son pH est ajusté à 4,5 avec de l'acide sulfurique 4N.

Après 30 minutes d'agitation, la solution est centrifugée et le précipité est recueilli, puis lavé par de l'eau à pH 4,5. Il est ensuite lyophilisé : on obtient une poudre dont la teneur en protéines est de 85,3 % en poids.

### Exemple 6 :

On ajoute, sous agitation dans un réacteur thermostaté, 600 g de farine délipidée enrichie dans 6 litres d'eau distillée contenant 5g/l de NaCl. Le pH mesuré est ajusté à 7,5.

La solution est pompée par l'intermédiaire d'une pompe péristaltique dans un tube ultrasonique à passage intégral (puissance des ultrasons 600 W - fréquence 20 000 Hz), le débit étant de 60 l/h.

Deux passages discontinus par charges successives sont réalisés (l'extrait est recueilli après passage dans le tube ultrasonique dans un second réacteur).

Après centrifugation, puis filtration sur 5µm, on obtient les résultats suivants :
- protéines de l'extrait après le premier passage : 14,2 g/l
- protéines de l'extrait après le deuxième passage : 15,2 g/l

### Exemple 7 :

On ajoute dans un réacteur thermostaté sous agitation, 300 g de farine délipidée enrichie dans 6 litres d'eau distillée contenant 5 g/l de NaCl. Le pH mesuré est ajusté en continu à 7,5.

La solution est introduite pendant une heure, en continu et en circuit fermé, dans le tube ultrasonique à passage intégral de l'exemple 6 (puissance des ultrasons : 700 W - fréquence : 20 000 Hz), le débit étant de 60 l/h.

Une circulation d'eau froide permet de maintenir la température de la solution dans la cuve à environ 33° C.

Des prélèvements sont effectués après 15 min, 30 min et 45 min d'extraction.

Après centrifugation, puis filtration sur 5 µm des extraits, on obtient les résultats suivants :

| Durée d'extraction (min) | Protéines dans l'extrait filtré Biuret (g/l) |
|---|---|
| 15 | 8,23 |
| 30 | 8,56 |
| 45 | 8,89 |
| 60 | 9,89 |

### Exemple 8 :

Dans un réacteur, on introduit 25,0 Kg d'eau distillée et on réalise successivement les opérations suivantes :
- disperser sous agitation 2,5 Kg de farine totale obtenue par broyage de graines d'Hibiscus entières,
- ajuster le pH à 9, avec NaOH 4N, après 15 minutes de dispersion,
- extraire sous agitation pendant 2 heures à température ambiante en maintenant le pH à 9 par ajout de NaOH 4N,
- centrifuger pendant 10 minutes à 5000 g,
- récupérer le surnageant beige trouble,
- ajuster le pH à 7,5 par addition de H2SO4 4N,
- centrifuger,
- clarifier par une nouvelle centrifugation,
- récupérer le surnageant opalescent,
- filtrer jusqu'à 0,5 µm,
- récupérer le surnageant,
- atomiser.

On peut alors récupérer une poudre beige clair avec un rendement d'atomisation de 62,7 % en poids.

### Exemple 9 :

En première variante de l'exemple 8, on peut également réaliser les opérations suivantes :
- ajuster le pH d'une partie de l'extrait préparé selon l'exemple 8 à 4,1 par addition de H2SO4 4N,
- laisser reposer à + 4° C (formation d'un précipité - environ 50 % du volume total),
- centrifuger à 5000 g pendant 15 mn,
- laver les culots avec de l'eau distillée à pH = 4,1,
- centrifuger à 5000 g pendant 15 mn,
- recueillir le précipité,
- reconstituer le précipité dans de l'eau distillée (10 % du volume avant précipitation),
- ajuster le pH à 7,5 en ajoutant sous agitation NaOH 4N,
- homogénéiser pour favoriser la solubilisation et l'homogénéisation,
- centrifuger pendant 10 min à 5000 g pour éliminer l'insoluble,
- atomiser le concentré protéique (rendement de l'atomisation sur la base de l'extrait sec : 82,90 % en poids).

### Exemple 10 :

En seconde variante de l'exemple 8, on peut réaliser les opérations suivantes :
- ajuster le pH d'une partie de l'extrait préparé selon l'exemple 8 à pH 5 par addition de H2SO4 4N,
- laisser reposer une heure à température ambiante,
- centrifuger,
- recueillir le précipité,
- reconstituer le précipité dans 10 % du volume avant précipitation sans effectuer l'étape de lavage,
- ajuster le pH à 7,5 en ajoutant sous agitation NAOH 4N,
- homogénéiser pour favoriser la solubilisation et l'homogénéisation,
- centrifuger pendant 10 min à 5000 g pour éliminer l'insoluble,
- atomiser le concentré protéique.

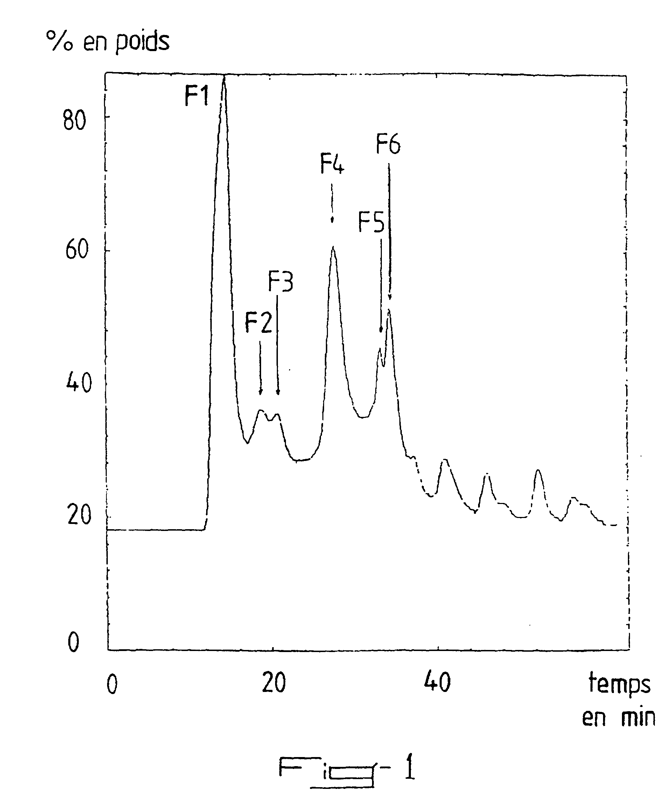

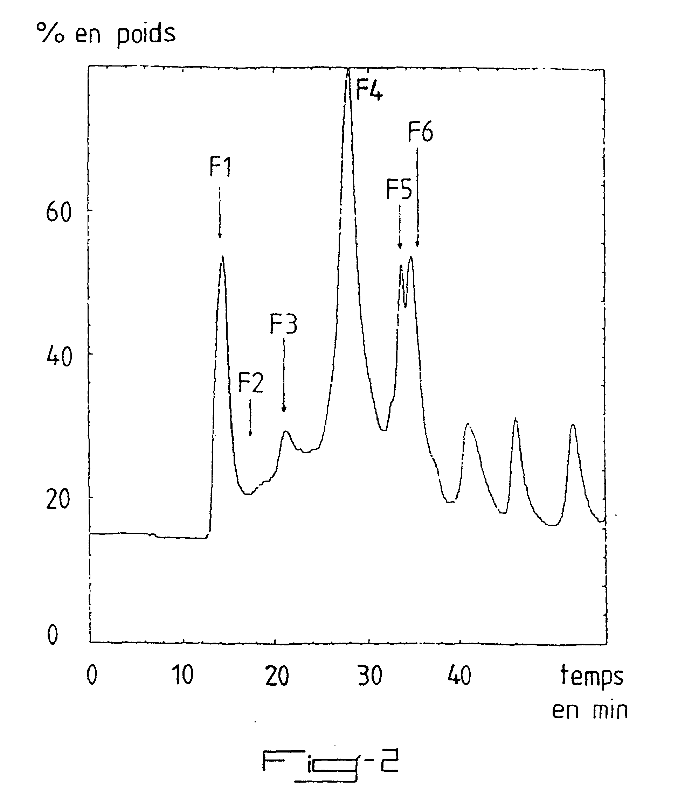

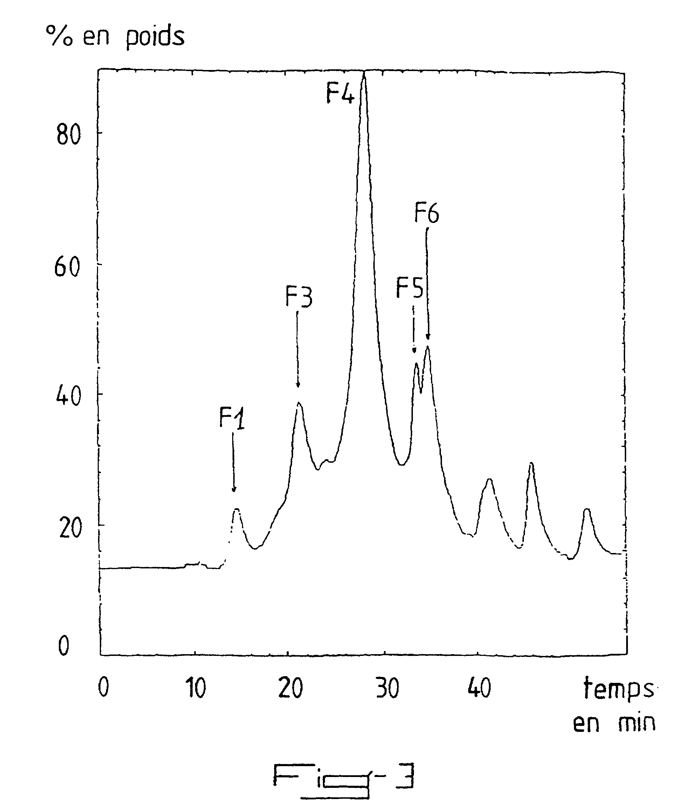

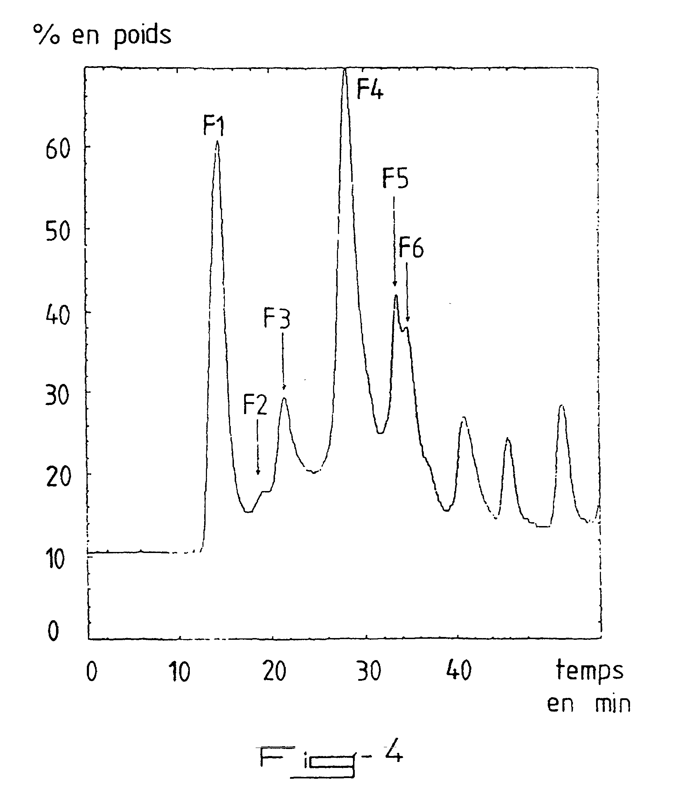

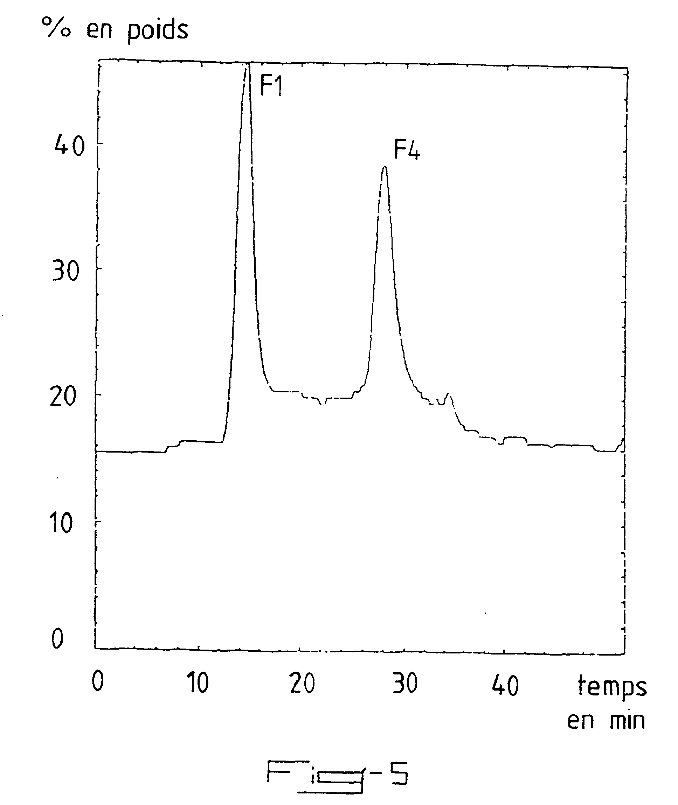

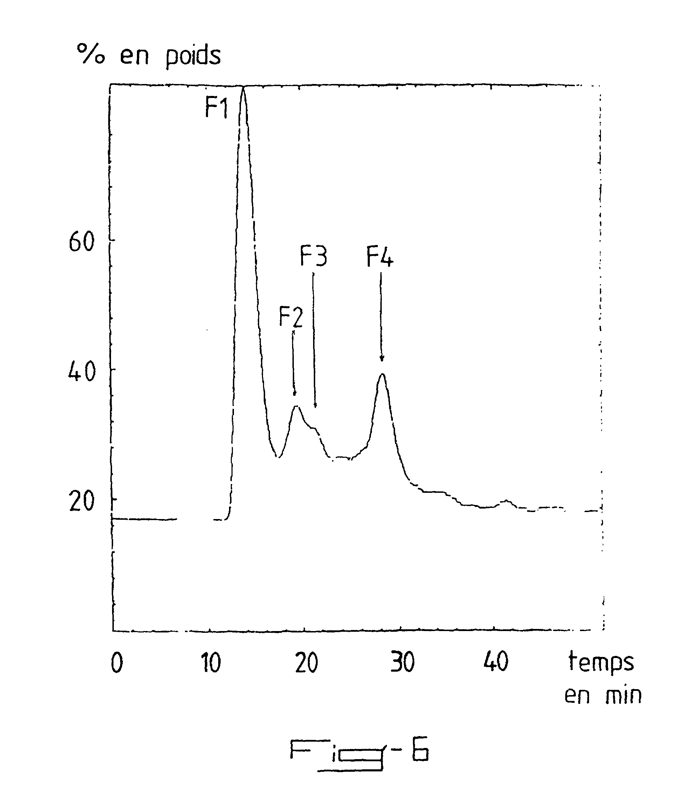

L'analyse en perméation de gel sur colonne du type superose 12 HR des fractions extraites par les différentes méthodes décrites ci-dessus permet de caractériser au moins 6 fractions différentes comme le montrent les diagrammes des figures 1 à 6 des dessins annexés, dans lesquels :
- la figure 1 représente la répartition des poids moléculaires des protéines d'un extrait de farine de graines entières d'Hibiscus esculentus (extraction : 2 heures à température ambiante dans l'eau, pH = 9),
- la figure 2 représente la répartition des poids moléculaires des protéines d'un extrait de farine de graines décortiquées d'Hibiscus esculentus (extraction : 1,5 heure à température ambiante dans l'eau, pH = 9),
- la figure 3 représente la répartition des poids moléculaires des protéines d'un extrait de farine de graines décortiquées d'Hibiscus esculentus (extraction : 1,5 heure à température ambiante dans du NaCl 10g/l, pH = 9),
- la figure 4 représente la répartition des poids moléculaires des protéines d'un extrait de farine de graines décortiquées d'Hibiscus esculentus (extraction dans un tube à ultrasons, 1 heure dans du NaCl 5g/l, pH = 7,5),
- la figure 5 représente la répartition des poids moléculaires des protéines d'un concentré protéique obtenu par précipitation à pH = 4,1 d'un extrait de farine de graines entières d'Hibiscus esculentus, et,
- la figure 6 représente la répartition des poids moléculaires des protéines d'un concentré protéique obtenu par précipitation à pH = 5 d'un extrait de farine de graines entières d'Hibiscus esculentus.

Le tableau récapitulatif suivant (en deux parties) montre la répartition des différentes fractions protéiques extraites.

| Conditions d'extraction | pH = 9 eau | pH = 9 NaCl 1g/l | pH = 9 NaCl 5g/l | pH = 9 NaCl 10g/l | pH = 6,5 NaCl 5g/l | pH = 8 NaCl 5g/l |
|---|---|---|---|---|---|---|
| | | | | | | 4h |
| PM > 500 000 Da | 13,7 | 9,8 | 4,4 | 3,7 | 2,9 | 2,7 |
| PM compris entre 100 000 et 500 000 Da | 12,2 | 11,2 | 10,7 | 14,4 | 9,7 | 17,3 |
| PM compris entre 30 000 et 100 000 Da | 4 | 3,3 | 7,6 | 7,7 | 14,7 | 7,9 |
| PM compris entre 5 000 et 30 000 Da | 41,2 | 44,7 | 46,3 | 51,2 | 49,6 | 45.9 |
| PM inférieur ou égal à 5 000 Da | 28,9 | 31 | 31 | 23 | 23,1 | 26,2 |

| Conditions d'extraction | pH = 7,5 NaCl 5g/l | pH = 7,5 NaCl 5g/l | pH = 7,5 NaCl 5g/l | pH = 9 H₂O | Concentré protéique | Concentré protéique |
|---|---|---|---|---|---|---|
| | 6 h | 15 min US | 60 min US | farine totale | (pH 4,1) | (pH 5) |
| PM > 500 000 Da | 4 | 13 | 20,9 | 30,5 | 35,5 | 46,6 |
| PM compris entre 100 000 et 500 000 Da | 13,1 | 14,2 | 3,8 | 15 | 12,2 | 17,5 |
| PM compris entre 30 000 et 100 000 Da | 4,3 | 6,1 | 15,1 | 5,9 | 8,1 | 7,3 |
| PM compris entre 5 000 et 30 000 Da | 50,2 | 46 | 35,3 | 26,4 | 35,4 | 22,6 |
| PM inférieur ou égal à 5 000 Da | 28,4 | 20,7 | 24,9 | 22,2 | 8,8 | 6 |

Les 6 fractions protéiques précitées sont constituées par :
- une fraction (notée Fl) de très haut poids moléculaire (entre 1 000 000 et 1 500 000 Da d'après l'étalonnage de la colonne),
- une fraction (notée F2) de poids moléculaire compris entre 250 000 et 350 000 Da surtout visible sur les extraits de farine de graines entières,
- une fraction (notée F3) de poids moléculaire compris entre 130 000 et 180 000 Da,
- une fraction (notée F4) qui correspond à un poids moléculaire compris entre 17 000 et 22 000 Da,
- deux fractions de poids moléculaires respectivement d'environ 3300 Da (F5) et d'environ 2300 à 2600 Da (F6).

Selon les conditions d'extraction, la fraction F1 constitue entre 2,7 et 50,00 % des protéines et la fraction F4 constitue entre 16 et 52 % (moyenne 40 %) des protéines totales.

On note que, plus le solvant d'extraction contient de sels au départ et moins la fraction F1 est importante.

Les extraits obtenus au moyen des exemples de procédés décrits sont directement utilisables sous forme liquide ou après séchage selon les techniques conventionnelles de déshydratation (atomisation, lyophilisation ...).

Les fractions protéiques extraites selon les exemples indiqués précédemment présentent l'avantage, par rapport à l'état naturel dans lequel elles se trouvent dans les graines, d'avoir une structure chimique native identique, de pouvoir être partiellement ou totalement purifiées (c'est-à-dire être débarassées des autres constituants des graines tels que les lipides, les fibres, les glucides ou analogues), et d'avoir une composition modulable en fonction du procédé d'extraction et/ou de purification mis en oeuvre (protéines totales de l'extrait brut, concentré protéique ou fraction(s) protéique(s) purifiée(s).

On relève, par rapport à la caséine lactique, une solubilité améliorée, en plus de l'origine végétale renouvelable recherchée dans le domaine cosmétique, ce tout en conservant une composition en acides aminés proche de la caséine dont on connaît les propriétés nutritionnelles, hydratantes et filmogènes pour la peau et les phanères.

Les fractions protéiques peuvent être utilisées soit sous leur forme native sans modification des structures initiales, soit sous la forme d'association(s) naturelle(s) de deux ou de toutes les fractions extraites correspondant aux différents pics des chromatogrammes représentés sur les dessins annexés, ou encore sous forme isolée, c'est-à-dire utilisation d'une ou de plusieurs fractions correspondant à un ou des pics des chromatogrammes précités.

Les fractions protéiques peuvent également être utilisées sous leur forme modifiée ou fonctionnalisée par l'un quelconque des traitements suivants :
- polymérisation,
- hydrolyse chimique des protéines d'Hibiscus.
- hydrolyse enzymatique des protéines d'Hibiscus. Dans ce but, des protéases provenant d'extraits d'origines animale, végétale, microbienne ou fongique peuvent être utilisées pour modifier les protéines d'Hibiscus : Pepsine, Trypsine, Chymotrypsine / Papaïne, Pronase, Bromelaïne / Endoprotéinase, Thermitase, Protéases de Bacillus subtilis, d'Aspergillus Niger et d'Aspergillus Oryzae / Subtilisine, Alcalase, Neutrase.
- transformation microbiologique avec utilisation des protéines d'Hibiscus en tant que substrat de fermentation par divers micro-organismes tels que des levures (Saccharomyces), des moisissures (du type Aspergillus), des bactéries telles que Bacillus ou analogues.
- fonctionnalisation chimique ou enzymatique par des procécés tels que la désamidation, la succinylation ou la phosphorylation.
- quaternisation.
- greffage de molécules saccharidiques ou lipidiques.

L'invention a également pour objet une composition cosmétique, notamment à application topique pour la peau et/ou les phanères, caractérisée en ce qu'elle contient en tant que substitut de caséine au moins une fraction protéique, préférentiellement soluble, extraite de graines d'Hibiscus esculentus.

Selon une caractéristique de l'invention, la ou les fraction(s) protéique(s) est (sont) extraite(s) de farine non délipidée ou délipidée de graines entières ou décortiquées d'Hibiscus esculentus par l'eau ou des solutions salines à différents pH.

Selon un mode de réalisation préférentiel de l'invention, la ou les fraction(s) protéique(s) est (sont) extraite(s) en solution aqueuse sous l'action d'ultrasons et cette ou ces fraction(s) protéique(s) est (sont) purifiée(s) par un procédé de purification choisi dans le groupe formé par la précipitation, l'adsorption, la chromatographie d'échange d'ions ou d'affinité et l'ultrafiltration.

La fraction protéique totale ou native au moins présente est choisie dans le groupe des fractions protéiques totales et natives extraites de graines d'Hibiscus esculentus et présentant en filtration sur gel des poids moléculaires apparents de 1 000 000 à 1 500 000 Da, 250 000 à 350 000 Da, 130 000 à 180 000 Da, 17 000 à 22 000 Da, 3 300 Da et 2 300 à 2 600 Da.

La ou les fraction(s) protéique(s) précitée(s) peut (peuvent) consister en un hydrolysat chimique ou enzymatique préparé à partir de protéines natives, être obtenue(s) par polymérisation ou dépolymérisation de protéines natives ou être chimiquement modifiée(s) par greffage.

Selon un premier mode de réalisation de l'invention, la composition cosmétique contient au moins deux fractions protéiques de poids moléculaires apparents différents.

Conformément à un second mode de réalisation de l'invention la composition cosmétique contient un extrait de graines d'Hibiscus esculentus constitué par l'ensemble des fractions protéiques solubles présentes naturellement dans ces graines.

Ladite composition cosmétique selon l'une des revendications 1 à 11, caractérisée en ce qu'elle comporte préférentiellement entre 0,01 % et 50,00 % en poids, préférentiellement entre 1 % et 25 % en poids, de fraction(s) protéique(s) extraite(s) de graines d'Hibiscus esculentus, telle(s) qu'obtenue(s) par l'un quelconque des exemples de procédés d'obtention décrits précédemment.

A titre d'exemples non limitatifs de réalisations pratiques de l'invention, on décrira ci-après différents produits ou compositions cosmétiques comprenant au moins une fraction protéique soluble extrait de graines d'Hibiscus esculentus ou Okra.

### Exemple 1 :

Un produit cosmétique conforme à l'invention, sous forme de crème de jour hydratante et adoucissante pour le visage et le corps pourra, par exemple, présenter une composition pondérale, constituée à partir des fractions A, B, C et D suivantes, telle qu'indiquée ci-après.
Fraction A :
   - Cutina MD 14,00 %
   - Eutanol G 6,00 %
   - Cétiol B 6,00 %
   - Eumulgin B1 1,50 %
   - Eumulgin B2 1,50 %
Fraction B :
   - Extraits de protéines totales natives d'Hibiscus selon l'exemple n° 1 précité 8,00 %
Fraction C :
   - Allantoine 0,20 %
   - Methylparaben 0,20 %
   - Germall 115 0,30 %
   - Eau distillée 62,00 %
Fraction D :
   - Parfum 0,30 %
Le procédé de préparation et de fabrication de la crème de jour précitée consiste essentiellement à chauffer la fraction A à 75° C, à préparer la fraction C à 75° C, à verser la fraction A dans la fraction B, sous agitation turbine, à ajouter la fraction C vers 50° C, puis à continuer l'agitation planétaire jusqu'à température ambiante et, enfin, à ajouter la fraction D.

### Exemple 2 :

Un produit cosmétique conforme à l'invention, sous forme de crème réparatrice restructurante, anti-rides et nourissante pour la nuit pourra, par exemple, présenter une composition pondérale, constituée à partir des fractions A, B, C et D suivantes, telle qu'indiquée ci-après.
Fraction A :
   - Miglyol 810 6,00 %
   - Myrj 51 3,00 %
   - Arlatone 983 S 2,00 %
   - Acide stéarique TP 4,00 %
   - Alcool cétylique 3,00 %
Fraction B :
   - Propylène Glycol 3,00 %
   - Elestab LS 388 (Laboratoires Serobiologiques) 2,50 %
   - Eau distillée 61,20 %
Fraction C :
   - Extraits atomisé de protéines natives d'Hibiscus selon l'exemple n° 9 précité 2,00 %
   - Eau distillée 13,00 %
Fraction D :
   - Parfum 0,30 %
   Le procédé de préparation et de fabrication de la crème de nuit précitée consiste essentiellement à préparer et à chauffer les fractions A et B à 75° C, à verser la fraction A dans la fraction B, sous agitation turbine, à préparer (séparément et extemporanément) le soluté de la fraction C par agitation de l'atomisat dans l'eau distillée, à ajouter la fraction C dans l'émulsion A + B en cours de refrodissement vers 50° C, puis à continuer en agitation planétaire à partir de 45° C et jusqu'à température ambiante, en ajoutant le parfum vers 40° C.

### Exemple 3 :

Un produit cosmétique conforme à l'invention, sous forme de microémulsion protectrice, conditionneuse, coiffante et photoprotectrice pour cheveux pourra, par exemple; présenter une composition pondérale, constituée à partir des fractions A, B, C et D suivantes, telle qu'indiquée ci-après.
Fraction A :
   - Brij 96 11,80 %
   - Arlatone G 10,00 %
   - Huile de paraffine 13,00 %
Fraction B :
   - Eau distillée 53,70 %
   - Méthylparaben 0,20 %
   - Elestab 4112 (Laboratoires Sérobiologiques) 0,30 %
Fraction C :
   - Protéines natives d'Hibiscus sous forme de concentré protéique selon l'exemple n° 6 précité 5,00 %
   - Eau distillée 5,00 %
Fraction D :
   - Parfum 0,20 %
   - Tween 20 0,80 %
Le procédé de préparation et de fabrication de la micro-émulsion précitée consiste essentiellement à préparer et à chauffer les fractions A et B à 75° C, à verser la fraction A dans la fraction B, sous agitation turbine, à réaliser un refroidissement progressif et, vers 50 ° C, à ajouter la fraction C puis la fraction D, et à poursuivre l'agitation jusqu'à refroidissement à température ambiante et homogénéisation parfaite.

## Revendications

1. Utilisation d'au moins une fraction protéique de graines d'Hibiscus esculentus ou Okra dans une composition ou un produit cosmétique.

2. Composition cosmétique **caractérisée en ce qu'**elle contient, en tant que substitut de caséine, entre 0,01 % et 50,00 % en poids de fraction(s) protéique(s) extraite(s) de graines d'Hibiscus esculentus.

3. Composition cosmétique selon la revendication 2, **caractérisée en ce que** la ou les fraction(s) protéique(s) est (sont) extraite(s) de farine non délipidée ou délipidée de graines entières ou décortiquées d'Hibiscus esculentus par l'eau ou des solutions salines à différents pH.

4. Composition cosmétique selon l'une quelconque des revendications 2 et 3, **caractérisée en ce que** la ou les fraction(s) protéique(s) est (sont) extraite(s) en solution aqueuse sous l'action d'ultrasons.

5. Composition cosmétique selon l'une quelconque des revendications 2 à 4, **caractérisée en ce que** la ou les fraction(s) protéique(s) est (sont) purifiée(s) par un procédé de purification choisi dans le groupe formé par la précipitation, l'adsorption, la chromatographie d'échange d'ions ou d'affinité et l'ultrafiltration.

6. Composition cosmétique selon l'une des revendications 2 à 5, **caractérisée en ce que** la fraction protéique totale ou native est choisie dans le groupe des fractions protéiques totales et natives extraites de graines d'Hibiscus esculentus et présentant en filtration sur gel des poids moléculaires apparents de 1 000 000 à 1 500 000 Da, 250 000 à 350 000 Da, 130 000 à 180 000 Da, 17 000 à 22 000 Da, 3 300 Da et 2 300 à 2 600 Da.

7. Composition cosmétique selon l'une quelconque des revendications 2 à 6, **caractérisée en ce que** la ou les fraction(s) protéique(s) consiste(nt) en un hydrolysat chimique ou enzymatique préparé à partir de protéines natives.

8. Composition cosmétique selon l'une quelconque des revendications 2 à 6, **caractérisée en ce que** la ou les fraction(s) protéique(s) est (sont) obtenue(s) par polymérisation de protéines natives.

9. Composition cosmétique selon l'une quelconque des revendications 2 à 7, **caractérisée en ce que** la ou les fraction(s) protéique(s) est (sont) chimiquement modifiée(s) par greffage.

10. Composition cosmétique selon l'une quelconque des revendications 2 à 9, **caractérisée en ce qu'**elle contient au moins deux fractions protéiques de poids moléculaires apparents différents.

11. Composition cosmétique selon l'une quelconque des revendications 2 à 9, **caractérisée en ce qu'**elle contient un extrait de graines d'Hibiscus esculentus constitué par l'ensemble des fractions protéiques solubles présentes naturellement dans ces graines.

## Patentansprüche

1. Verwendung mindestens einer Proteinfraktion aus Hibiscus-esculentus-(auch: Okra-)Samen in einer Kosmetikzusammensetzung oder einem Kosmetikprodukt.

2. Kosmetikzusammensetzung, **dadurch gekennzeichnet, daß** sie als Caseinersatz 0,01 Gew.-% bis 50,00 Gew.-% einer aus Hibiscus-esculentus-Samen extrahierten Proteinfraktion bzw. von aus Hibiscus-esculentus-Samen extrahierten Proteinfraktionen enthält.

3. Kosmetikzusammensetzung nach Anspruch 2, **dadurch gekennzeichnet, daß** die Proteinfraktion(en) aus gegebenenfalls entfettetem Mehl von ganzen oder entschalten Hibiscus-esculentus-Samen mit Wasser oder mit Salzlösungen bei unterschiedlichen pH-Werten extrahiert wird bzw. werden.

4. Kosmetikzusammensetzung nach Anspruch 2 oder 3, **dadurch gekennzeichnet, daß** die Proteinfraktion(en) mit einer wäßrigen Lösung unter Einwirkung von Ultraschall extrahiert wird bzw. werden.

5. Kosmetikzusammensetzung nach einem der Ansprüche 2 bis 4, **dadurch gekennzeichnet, daß** die Proteinfraktion(en) mit einem Reinigungsverfahren aus der Gruppe Fällung, Adsorption, Ionenaustauschchromatographie, Affinitätschromatographie und Ultrafiltration gereinigt wird bzw. werden.

6. Kosmetikzusammensetzung nach einem der Ansprüche 2 bis 5, **dadurch gekennzeichnet, daß** die Gesamtproteinfraktion bzw. die Fraktion nativen Proteins aus der Gruppe der aus Hibiscus-esculentus-Samen extrahierten Gesamtproteinfraktionen und Fraktionen der nativen Proteine ausgewählt wird, die bei Gelfiltration scheinbare Molekulargewichte von 1 000 000 bis 1 500 000 Da, 250 000 bis 350 000 Da, 130 000 bis 180 000 Da, 17 000 bis 22 000 Da und 3 300 bis 2 600 Da aufweisen.

7. Kosmetikzusammensetzung nach einem der Ansprüche 2 bis 6, **dadurch gekennzeichnet, daß** die Proteinfraktion(en) aus einem aus nativen Proteinen hergestellten chemischen oder enzymatischen Hydrolysat besteht bzw. bestehen.

8. Kosmetikzusammensetzung nach einem der Ansprüche 2 bis 6, **dadurch gekennzeichnet, daß** die Proteinfraktion(en) durch Polymerisation von nativen Proteinen erhalten wird bzw. werden.

9. Kosmetikzusammensetzung nach einem der Ansprüche 2 bis 7, **dadurch gekennzeichnet, daß** die Proteinfraktion(en) chemisch durch Pfropfung modifiziert wird bzw. werden.

10. Kosmetikzusammensetzung nach einem der Ansprüche 2 bis 9, **dadurch gekennzeichnet, daß** sie mindestens zwei Proteinfraktionen mit unterschiedlichen scheinbaren Molekulargewichten enthält.

11. Kosmetikzusammensetzung nach einem der Ansprüche 2 bis 9, **dadurch gekennzeichnet, daß** sie einen Hibiscus-esculentus-Samenextrakt enthält, der aus allen natürlich in diesen Samen vorliegenden löslichen Proteinfraktionen besteht.

## Claims

1. Use of at least one protein fraction from *Hibiscus esculentus* or okra seeds in a cosmetic composition or product.

2. Cosmetic composition, **characterised in that** it contains, as a substitute for casein, between 0.01 % and 50.00 % by weight of protein fraction(s) extracted from Hibiscus esculentus seeds.

3. Cosmetic composition according to claim 2, **characterised in that** the protein fraction(s) is (are) extracted from non-delipidated or delipidated flour of whole or dehusked *Hibiscus esculentus* seeds by water or saline solutions at various pH.

4. Cosmetic composition according to any one of claims 2 and 3, **characterised in that** the protein fraction(s) is (are) extracted in aqueous solution under the influence of ultrasound.

5. Cosmetic composition according to any one of claims 2 to 4, **characterised in that** the protein fraction(s) is (are) purified by a purification process selected from the group formed by precipitation, adsorption, ion or affinity exchange chromatography and ultrafiltration.

6. Cosmetic composition according to one of claims 2 to 5, **characterised in that** the total or native protein fraction is selected from the group of total and native protein fractions extracted from *Hibiscus esculentus* seeds and having apparent molecular weights when filtered over gel of 1,000,000 to 1,500,000 Da, 250,000 to 350,000 Da, 130,000 to 180,000 Da, 17,000 to 22,000 Da, 3,300 Da and 2,300 to 2,600 Da.

7. Cosmetic composition according to any one of claims 2 to 6, **characterised in that** the protein fraction(s) consist(s) of a chemical or enzymatic hydrolyzate prepared from native proteins.

8. Cosmetic composition according to any one of claims 2 to 6, **characterised in that** the protein fraction(s) is (are) obtained by polymerisation of native proteins.

9. Cosmetic composition according to any one of claims 1 to 7, **characterised in that** the protein fraction(s) is (are) chemically modified by grafting.

10. Cosmetic composition according to any one of claims 2 to 9, **characterised in that** it contains at least two protein fractions having different apparent molecular weights.

11. Cosmetic composition according to any one of claims 2 to 9, **characterised in that** it contains an extract of *Hibiscus esculentus* seeds made up of all the soluble protein fractions naturally present in these seeds.
